# EUROPEAN PATENT SPECIFICATION

(11) **EP 0 559 775 B1**
(45) Date of publication and mention of the grant of the patent: **28.08.1996**
(21) Application number: 92901245.8
(22) Date of filing: 19.11.1991
(51) Int. Cl.: G05D 16/00, A61L 2/00, B65D 51/16

(54) **CONTACT LENS CASE VENTING SYSTEM**
LÜFTUNGSSYSTEM EINES BEHÄLTERS FÜR KONTAKTLINSEN
SYSTEME DE MISE A L'AIR DE FLACON POUR LENTILLES DE CONTACT

(30) Priority: 30.11.1990 US 621351
(43) Date of publication of application: 15.09.1993
(73) Proprietor: CIBA VISION CORPORATION, Atlanta, GA 30360 (US)
(72) Inventor: KANNER, Rowland, W., Guntersville, AL 35976 (US)
(74) Representative: Long, Edward Anthony
(86) International application number: US9108666
(87) International publication number: WO9209942

(56) References cited:
- GB-A- 2 209 845
- US-A- 3 527 376
- US-A- 4 396 583
- US-A- 4 637 919
- US-A- 4 672 996
- US-A- 4 750 610
- US-A- 4 889 693
- US-A- 4 956 156

## Description

### Background of the Invention

This invention relates to container appliances for chemical sterilization of small articles such as contact lenses, and more particularly, relates to pressure relief venting of such appliances.

The well-known, commercialized contact lens disinfection process employing hydrogen peroxide solution as a bactericide is described for example in U.S. Patent No.s 4,750,610; 4,889,693; and 4,956,156. In such process, the contact lenses are immersed overnight in an aqueous solution of hydrogen peroxide which is catalytically decomposed during the sterilization process resulting in liberation of oxygen gas. As a result, the liberated oxygen produces a pressure increase within the disinfecting vessel which is accordingly provided with venting structure for relief of the pressure to the ambience. Further, in US 3,527,376 is described an arrangement in which a diaphragm is clamped between upper and lower portions of a cap and seats in a recess formed in the lower portion of the cap.

The present invention provides simplified reliable, venting structure in such pressurized vessels particularly for low pressure discharge of the gas. The invention would of course be usable with any disinfecting process wherein a gaseous material is liberated during the process.

### Summary of the Invention

In accordance with the present invention, there is provided an appliance for disinfecting contact lenses or the like wherein the lenses are disposed within a disinfecting solution which liberates a gas during the disinfecting process including a normally closed vent conduit for passage of pressurized effluent gas from a container body through a removable cap; and
a check valve in said normally closed vent conduit including a disc member having a slit therethrough which opens for vent discharge of said pressurized effluent gas by sufficient pressure on said disc member exerted by said pressurized effluent gas during passage through said normally closed vent conduit, after which said slit recloses to prevent any subsequent leakage of any disinfecting solution therethrough, CHARACTERIZED IN THAT said cap includes a supporting surface against which said closed slit is engaged when closed, and from which said slit disengages when opened to form a one-way venting valve.

Further and preferred features of the invention are as follows.

The cap may comprise upper and lower portions and wherein said support surface is formed within a recess in said lower cap portion. The disc member may be clamped between a supporting shoulder formed in said lower cap portion and securing projection from said upper cap portion. The securing projection may comprise a ring extending from said upper cap portion. The lower cap portion may include at least one through passageway having one end opening against said disc member and a second end opening to communicate with said pressurized effluent gas within said container body to form a portion of said vent conduit enabling said exertion of said pressurized effluent gas against said disc member to open said slit. The lower cap portion may include a plurality of through passageways opening against said disc member respectively spaced from said slit. The disc member may have a generally circular configuration and said slit linearly aligned along a diameter of said disc member at a generally central location, and said plurality of through passageways may be arranged to open against said disc member in a radially symmetrical pattern relative to said slit. The appliance may further comprise lens holder means for supporting contact lenses within said container body.

### Brief Description of the Drawings

FIG. 1 is a sectional view of one embodiment of a lens sterilizing appliance in accordance with the present invention;
FIG. 2 is a fragmentary sectional view taken from FIG. 1 illustrating passage of gas through an opened check valve formed in the cap of the appliance; and
FIG. 3 is a plan view of the check valve disc shown in FIGS. 1 and 2.

### Detailed Description of the Illustrated Embodiment

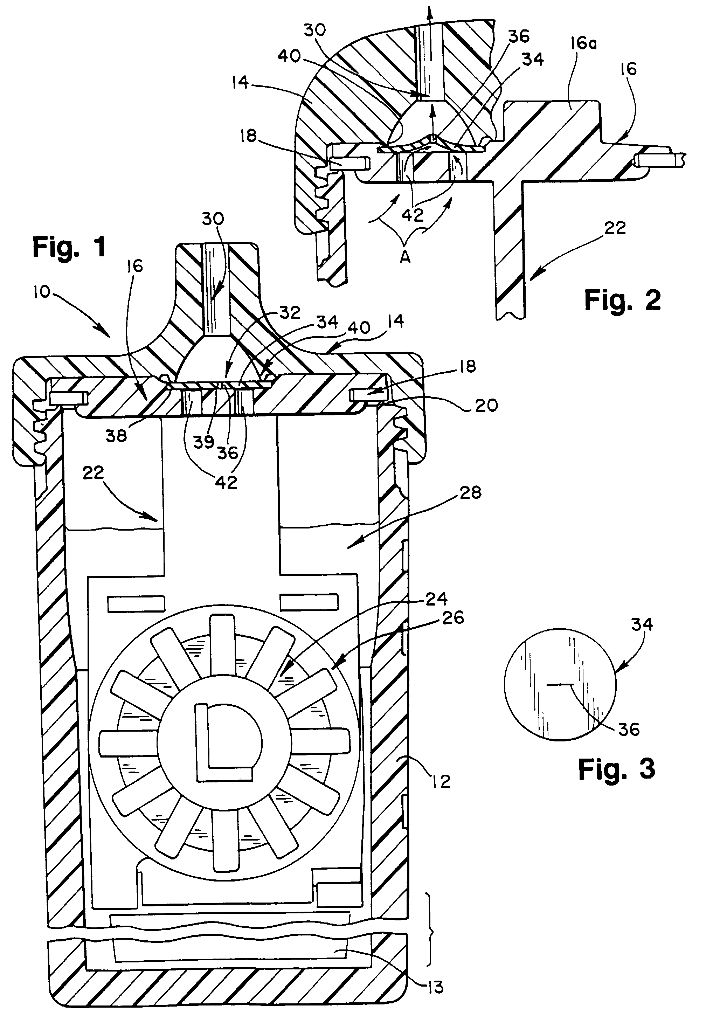

Referring now in more detail to FIGS. 1 and 2, one embodiment of the contact lens disinfecting appliance in accordance with the present invention is generally designated by reference character 10. The appliance 10 includes a generally cylindrical container body 12 having a threaded end opening for receiving a removable screw cap 14. As best shown in FIGs. 1 and 2, a lower portion of cap 14 includes a closure flange 16 having an upwardly projecting tongue 16a which is secured in a mating mortise, for example, by sonic welding. The closure disc or flange 16 has a grooved periphery within which an annular gasket 18 is secured. The gasket 18 seals against the end rim 20 at the opening of the container body 12. A depending lens-supporting frame 22 is secured to and projects downwardly into the cavity within the cylindrical container body 12 when the cap 14 is mounted thereon. A pair of contact lenses 24 are enclosed and supported by the frame 22 and respective pivotal baskets 26 (one of which is illustrated) as more fully described in U.S. Patent No. 4,750,610, the text of which is incorporated by reference herein. The baskets 26 are perforated to allow passage of the disinfecting liquid such as hydrogen peroxide solution 28 within which the lenses 24 are immersed.

In the disinfecting process, the hydrogen peroxide solution is poured into the container body 12 and the cap 14 is threaded thereon to seal the gasket 18 against the rim 20 as shown in FIG. 3, thereby immersing the lenses 24 within the solution. A catalytic element 13 initiates decomposition of the hydrogen peroxide solution and as the disinfecting reaction proceeds, the liberated oxygen exerts progressively elevated pressure within the container body 12.

In order to vent the elevated pressure generated within the container body 12, the cap 14 is provided with a vent passageway 30 which opens through the top of the cap 14 and a venting check valve structure generally designated by reference character 32 which leads from the interior of the container body to the vent passageway 30. The check valve structure 32 includes a valve disc 34 with a generally centrally located slit 36 as best shown in FIG. 3. The disc 34 is seated on a recess 38 formed within the cap flange 16. As best shown in FIG. 2, the vent structure 32 and passageway 30 are offset in relation to the centrally positioned lens support frame 22. The disc 34 is peripherally clamped against the flange recess 38 by an overlying ring 40 which is molded to downwardly project from the upper portion of the cap 14.

As best shown in FIG. 1, the normally closed valve slit 36 is seated upon the central surface 39 of the seating recess 38 which also has a plurality of vent ports 42 which pass through the cap flange 16 and open against the valve disc 34 spaced from the valve slit 36.

When the elevated pressure of the liberated oxygen gas is communicated through the ports 42 against the disc 34 as shown in FIG. 2, the upwardly directed gas flow indicated by arrows A will lift and upwardly deflect the central portion of the disc 34 to open the slit 36 allowing passage of the gas upwardly through the open slit leading to the passageway 30 and discharge therefrom to the ambience. Following gas discharge to sufficiently reduce the pressure within the container 12, the deflected central portion of the disc 34 will downwardly redeflect to reclose the slit 36 against the surface 39 of the recess 38 again in the position shown in FIG. 1 so that the closed slit will prevent any subsequent leakage of solution or entry of air. The slitted disc 34 functions as a one-way check valve and allows only vent discharge of pressurized gas. As a result, the closed slit also prevents any backflow and entry of bacterial or other contamination into the interior of the container, while automatically opening to vent subsequently generated excessive gas pressure.

The valve disc 34 can be fabricated, for example, from typical elastomeric composition such as silicone rubber. In typical lens sterilization containers, a silicone rubber disc in a grade of 50 durometer and dimensioned approximately 0.30 inch in diameter and 0.030 inch thick wit a slit of approximately 0.060 inch has performed to open at a threshold pressure in the range of approximately 6-8 psig and enabled a vent gas flow rate of approximately 30 cc's per minute at pressures of approximately 25 psig.

## Claims

1. An appliance (10) for disinfecting contact lenses (24) or the like wherein the lenses are disposed within a disinfecting solution (28) which liberates a gas (A) during the disinfecting process including a normally closed vent conduit (30,42) for passage of pressurized effluent gas (A) from a container body (12) through a removable cap (14); and
a check valve (32) in said normally closed vent conduit including a disc member (34) having a slit (36) therethrough which opens for vent discharge of said pressurized effluent gas by sufficient pressure on said disc member exerted by said pressurized effluent gas during passage through said normally closed vent conduit, after which said slit (36) recloses to prevent any subsequent leakage of any disinfecting solution (28) therethrough, CHARACTERIZED IN THAT said cap (14) includes a supporting surface (39) against which said closed slit (36) is engaged when closed, and from which said slit (36) disengages when opened (Fig. 2) to form a one-way venting valve.

2. An appliance according to claim 1, wherein said cap comprises upper and lower portions and wherein said support surface is formed within a recess (38) in said lower cap portion.

3. An appliance according to claim 2, wherein said disc member (34) is clamped between a supporting shoulder formed in said lower cap portion and a securing projection (40) from said upper cap portion.

4. An appliance according to claim 3, wherein said securing projection (40) comprises a ring extending from said upper cap portion.

5. An appliance according to claim 1, wherein said lower cap portion includes at least one through passageway (42) having one end opening against said disc member (34) and a second end opening to communicate with said pressurized effluent gas within said container body (12) to form a portion of said vent conduit (30,42) enabling said exertion of said pressurized effluent gas against said disc member (34) to open said slit (36).

6. An appliance according to claim 5, wherein said lower cap portion includes a plurality of through passageways (42) opening against said disc member (34) respectively spaced from said slit (36).

7. An appliance according to claim 6, wherein said disc member (34) has a generally circular configuration and said slit (36) is linearly aligned along a diameter of said disc member (34) at a generally central location, and said plurality of through passageways (42) are arranged to open against said disc member (34) in a radially symmetrical pattern relative to said slit (36).

8. An appliance according to claim 1, further comprising lens holder means for supporting contact lenses within said container body (12).

## Patentansprüche

1. Vorrichtung (10) zum Sterilisieren von Kontaktlinsen (24) oder ähnlichen Gegenständen, wobei die Linsen in einer desinfizierenden Lösung (28) aufbewahrt werden, die während des Sterilisierungsvorgangs ein Gas (A) freisetzt, und wobei die Vorrichtung einen im Normalfall geschlossenen Entlüftungskanal (30, 42) beinhaltet, durch den das unter Druck stehende Gas (A) aus dem Behälter (12) durch eine Schraubkappe (14) abströmt; und ebenfalls ein Rückschlagventil (32) in dem besagten und im Normalfall geschlossenen Entlüftungskanal mit einem Ventilteller (34) beinhaltet, der einen Schlitz (36) aufweist, der sich bei Erreichen eines bestimmten Druckgrenzwertes des innerhalb des im Normalfall geschlossenen Entlüftungskanals befindlichen Gases auf dem Ventilteller öffnet und das Gas nach außen abführt; und wonach sich der besagte Schlitz (36) wieder schließt und auf diese Weise verhindert wird, daß die desinfizierende Lösung (28) durch den Schlitz nach außen treten kann, **dadurch gekennzeichnet,** daß die besagte Kappe (14) eine Stützfläche (39) aufweist, auf der der besagte Schlitz (36) im geschlossenen Zustand liegt, und von der der besagte Schlitz (36) sich abhebt, wenn er sich öffnet (Fig. 2), wodurch ein Einwege-Druckausgleichsventil dargestellt wird.

2. Vorrichtung nach Anspruch 1, wobei die besagte Kappe aus einem oberen und einem unteren Teil besteht und die besagte Stützfläche in einer Aussparung (38) des besagten unteren Kappenteils geformt wird.

3. Vorrichtung nach Anspruch 2, wobei besagter Ventilteller (34) zwischen einer Auflagefläche als Teil des besagten unteren Kappenteils und einem Auflagering (40) als Teil des besagten oberen Kappenteils eingespannt ist.

4. Vorrichtung nach Anspruch 3, wobei besagter Auflagering (40) eine ringförmige Ausbildung des oberen Kappenteils ist.

5. Vorrichtung nach Anspruch 1, wobei besagter unterer Kappenteil mindestens einen durchgehenden Kanal (42) beinhaltet, dessen eine Öffnung in Richtung des besagten Ventiltellers (34) und die andere Öffnung in Richtung des Behälters zeigt und das unter Druck stehende Gas auf diese Weise nach Druck auf dem Ventilteller (34) und nach Öffnen des Schlitzes (36) über die Entlüftungskanäle (30, 42) abströmen kann.

6. Vorrichtung nach Anspruch 5, wobei besagter unterer Kappenteil eine Reihe von Entlüftungskanälen (42) beinhaltet, die nach oben gegen den Ventilteller (34) öffnen und sich in einem bestimmten Abstand vom Schlitz (36) befinden.

7. Vorrichtung nach Anspruch 6, wobei besagter Ventilteller (34) in allgemeinen kreisförmig ausgebildet ist und der Schlitz über einen im allgemeinen im Mittelpunkt des Kreis befindlichen Teil des Durchmessers verläuft, und wobei die besagten Entlüftungskanäle (42) so angeordnet sind, daß sie sich in einer in Relation zum besagten Schlitz (36) radialsymmetrischen Anordnung in Richtung des besagten Ventiltellers (34) öffnen.

8. Vorrichtung nach Anspruch 1, weiterhin bestehend aus einer Linsenhalterung zum Halten der Kontaktlinsen innerhalb des besagten Behälters (12).

## Revendications

1. Dispositif (10) pour désinfecter des lentilles de contact (24) ou similaires, selon lequel les lentilles sont placées à l'intérieur d'une solution désinfectante (28) qui libère un gaz (A) au cours du processus de désinfection, comprenant un conduit de décharge (30, 42), normalement fermé, prévu pour laisser passer le gaz usé sous pression (A) provenant d'un corps (12) de récipient, à travers un couvercle amovible (14); et
un clapet de non retour (32), dans ledit conduit de décharge normalement fermé, comprenant un élément formant disque (34) dans lequel est ménagée une fente (36) qui s'ouvre pour assurer la décharge dudit gaz usé sous pression lorsqu'une pression suffisante est exercée sur ledit élément formant disque par ledit gaz usé sous pression, lors de son passage dans ledit conduit de décharge normalement fermé, après quoi ladite fente (36) se referme pour éviter toute fuite ultérieure de la solution désinfectante (28) à travers cette dernière, CARACTERISE EN CE QUE ledit bouchon (14) comprend une surface de support (39) avec laquelle ladite fente (36) fermée vient en engagement lors de sa fermeture, et dont ladite fente (36) se dégage lors de son ouverture (figure 2) pour former un clapet de décharge à une voie.

2. Dispositif selon la revendication 1, caractérisé en ce que ledit bouchon comprend des parties supérieure et inférieure, et en ce que ladite surface de support est formée dans un évidement (38) ménagé dans ladite partie de bouchon inférieure.

3. Dispositif selon la revendication 2, caractérisé en ce que ledit élément formant disque (34) est serré entre un épaulement de support formé dans ladite partie de bouchon inférieure et une projection de fixation (40) s'avançant à partir de ladite partie de bouchon supérieure.

4. Dispositif selon la revendication 3, caractérisé en ce que ladite projection de fixation (40) comprend une bague s'étendant à partir de ladite partie de bouchon supérieure.

5. Dispositif selon la revendication 1, caractérisé en ce que ladite partie de bouchon inférieure comprend au moins un passage traversant (42) dont une extrémité s'ouvre contre ledit élément formant disque (34) et une deuxième extrémité s'ouvre pour communiquer avec ledit gaz usé sous pression à l'intérieur dudit corps (12) de récipient, pour former une partie dudit conduit de décharge (30, 42) permettant ladite application dudit gaz usé sous pression contre ledit élément formant disque (34) pour ouvrir ladite fente (36).

6. Dispositif selon la revendication 5, caractérisé en ce que ladite partie de bouchon inférieure comprend une pluralité de passages traversants (42) s'ouvrant contre ledit élément formant disque (34) respectivement espacé de ladite fente (36).

7. Dispositif selon la revendication 6, caractérisé en ce que ledit élément formant disque (34) présente une configuration généralement circulaire et ladite fente (36) est alignée, de manière linéaire, le long d'un diamètre dudit élément formant disque (34) au niveau d'un emplacement généralement central, et ladite pluralité de passages traversants (42) est disposée pour s'ouvrir contre ledit élément formant disque (34) selon une configuration radialement symétrique par rapport à ladite fente (36).

8. Dispositif selon la revendication 1, caractérisé en ce qu'il comprend, en outre, un moyen formant support de lentilles pour supporter les lentilles de contact à l'intérieur dudit corps (12) de récipient.
